# FASCICULE DE BREVET EUROPEEN

(11) **EP 0 759 740 B1**
(45) Date de publication et mention de la délivrance du brevet: **08.08.2001**
(21) Numéro de dépôt: 95920137.7
(22) Date de dépôt: 11.05.1995
(51) Int. Cl.: A61K 7/48, A61K 7/16

(54) **UTILISATION DE LA LAMINARINE ET DES OLIGOSACCHARIDES DERIVES DE LAMINARINE EN COSMETOLOGIE ET POUR LA FABRICATION D'UN MEDICAMENT DESTINE AU TRAITEMENT DE LA PEAU**
VERWENDUNG DES LAMINARINS ODER ABGELEITETEN OLIGOSACCHARIDEN IN DER KOSMETIK ODER DERMATOLOGIE
USE OF LAMINARIN AND OLIGOSACCHARIDES DERIVED THEREFROM IN COSMETICS AND FOR PREPARING A SKIN TREATMENT DRUG

(30) Priorité: 11.05.1994 FR 9405795
(43) Date de publication de la demande: 05.03.1997
(73) Titulaire: LABORATOIRES GOEMAR S.A., 35413 Saint-Malo (FR)
(72) Inventeur: YVIN, Jean-Claude, 35400 Saint-Malo (FR); LEVASSEUR, Florence, 35400 Saint-Malo (FR); HUD'HOMME, Fabienne, 22750 Saint-Jacut-de-la-Mer (FR)
(74) Mandataire: Hubert, Philippe
(86) Numéro de dépôt international: FR9500618
(87) Numéro de publication internationale: WO9531177

(56) Documents cités:
- EP-A- 0 470 872
- FR-A- 2 114 989
- CHEMICAL ABSTRACTS, vol. 116, no. 10, 9 Mars 1992, Columbus, Ohio, US; abstract no. 91169, & SU,A,1 644 961 (YU. A. FEDOROV ET AL.)
- CHEMICAL ABSTRACTS, vol. 116, no. 4, 27 Janvier 1992, Columbus, Ohio, US; abstract no. 27870, & SU,A,1 644 962 (YU. A. FEDOROV ET AL.)
- CHEMICAL ABSTRACTS, vol. 116, no. 4, 27 Janvier 1992, Columbus, Ohio, US; abstract no. 27871, & SU,A,1 644 963 (YU. A. FEDOROV ET AL.)

## Description

La présente invention concerne essentiellement l'utilisation de la laminarine et des oligosaccharides dérivés de laminarine en cosmétologie et pour la fabrication d'un médicament destiné au traitement de la peau.

On sait que la laminarine est un polymère de réserve des algues brunes constitué de polysaccharides de structures légèrement différentes selon la nature de l'algue.

D'une façon générale, la laminarine présente une masse moléculaire inférieure à environ 30000 daltons et est constituée de 20 à 60 unités D-glucopyranoside réparties selon une chaîne linéaire principale, dans laquelle ces unités sont reliées par des liaisons β(1-3) et des ramifications reliées à cette chaîne principale par des liaisons β(1-6).

Certaines de ces chaînes comportent une unité terminale réductrice constituée par une unité mannitol. On note également l'existence d'unités mannose au sein de cette structure.

La laminarine est généralement extraite d'algues marines macrophytes brunes de type phéophycées et en particulier des Fucales ou des Laminariales.

Diverses méthodes d'extraction peuvent être utilisées pour obtenir la laminarine.

On se reportera par exemple à celle décrite par Black et al. Appl. Chem. (1951), Volume 1, pages 505 à 517.

D'une façon plus générale, la laminarine peut être obtenue à partir d'algues brunes par tout procédé d'extraction permettant d'éliminer successivement les constituants autres que la laminarine (polysaccharides pariétaux, sels, etc..).

Ces procédés font notamment appel à des étapes de broyage, de précipitations en milieu acide ou basique, d'ultrafiltrations et de dialyses.

La laminarine est également commercialisée par exemple par la Société SIGMA Chimie SARL

Diverses publications scientifiques décrivent des propriétés élicitrices de la laminarine, suggérant son utilisation pour augmenter les réactions de défense des plantes.

Ainsi, NETZER et Al. révèlent qu'une infection par le pathogène S. oxysporum déclenche l'induction de laminarinase (Biological abstracts, Vol. 68, n° 1, 1979).

De même, BONHOFF et Al. révèlent les propriétés de la laminarine en tant qu'éliciteur de phytoalexine et de callose (Biological abstracts, Vol. 86, n° 4, 1988).

Par ailleurs, KUROSAKI et Al. (Biological abstracts, Vol. 85, n° 2, 1988) et PEARCE (Biological abstracts, Vol. 74, n° 8, 1982) confirment les effets éliciteurs de la laminarine, en particulier vis-à-vis de la lignification, tout en précisant que ces effets sont faibles comparés à ceux des éliciteurs connus.

La demande de brevet français No. 9208387 des demandeurs confirme les propriétés élicitrices de la laminarine et son utilité pour augmenter les réactions de défense des plantes, et révèle en outre que la laminarine présente des propriétés d'éliciteur de l'α-amylase à l'origine d'une activité d'accélérateur de la germination des graines et de la croissance des plantes.

On sait par ailleurs que la laminarine sulfatée possède des propriétés pharmacologiques intéressantes en particulier anti-coagulantes et hypocholestérolémiantes. (K.C. Guven et al., Introduction to Applied Physicology, 1990, pages 67 à 92).

Il a été découvert, et ceci constitue le fondement de la présente invention, que la laminarine, les oligosaccharides dérivés de laminarine et les compositions en contenant présentent des effets stimulants, régénérants, revitalisants et énergétiques sur les fibroblastes du derme humain, tissu de soutien de la peau et sur les kératinocytes d'épiderme cutané humain, cellules cibles des produits cosmétiques ou pharmaceutiques, notamment dermatologiques.

Ces effets ont été démontrés par l'étude de la stimulation de la néosynthèse des protéines dans des cultures de cellules de la peau.

Les cultures de cellules permettent en effet de mesurer la tolérance et l'activité de produits à tester, dans la mesure où, dans des conditions appropriées de culture, ces cellules conservent des fonctions proches de celles exprimées in vivo.

Il semble, sans que ceci constitue une interprétation théorique, que les effets de stimulation obtenus soient dûs aux deux mécanismes suivants:
- d'une part, la laminarine, les oligosaccharides dérivés de laminarine, et les compositions en contenant auraient un effet nutritif et apporteraient aux cellules des éléments absents ou présents en quantité insuffisante dans le milieu de culture ;
- d'autre part, ces produits auraient un effet semblable à ceux d'une hormone ("hormone like"), c'est-à-dire qu'ils transmettraient aux cellules des signaux qui modifieraient l'activité de ces dernières.

Il a été constaté que les effets de stimulation de la laminarine, des oligosaccharides dérivés de laminarine et des compositions en contenant, se manifestent à des concentrations faibles, compatibles avec celles qui peuvent être utilisées dans une formulation cosmétique ou pharmaceutique.

Ainsi, selon un premier aspect, la présente demande vise à couvrir l'utilisation en cosmétologie de la laminarine et des oligosaccharides dérivés de laminarine comme agent stimulant, régénérant, revitalisant et à activité énergétique sur les fibroblastes et les kératinocytes.

L'expression "oligosaccharides dérivés de laminarine" utilisée dans le cadre de la présente description et des revendications, désigne tout produit susceptible d'être obtenu à partir de la laminarine par un procédé comportant son hydrolyse chimique ou enzymatique.

L'hydrolyse chimique sera généralement réalisée par action sur la laminarine, d'un acide comme par exemple l'acide sulfurique 0,1 M à une température comprise entre 50°C et 100°C et de préférence d'environ 80°C, pendant une durée comprise entre 3 heures et 8 heures, de préférence 6 heures.

Cette hydrolyse sera éventuellement suivie d'une neutralisation par addition d'une base forte jusqu'à l'obtention d'un pH compris entre 6 et 8 puis d'une étape de dessalage par passage du produit ainsi neutralisé sur une résine échangeuse d'ions et éventuellement par une étape de réduction sous forme de poudre par lyophilisation ou atomisation.

L'hydrolyse enzymatique sera généralement réalisée par action, sur la laminarine, d'une enzyme de type Endo β-giucanase telle que la laminarinase qui peut être d'origine animale, végétale, bactérienne ou fongique.

Les paramètres de l'hydrolyse (pH, température, durée, concentrations du substrat et de l'enzyme) pourront être facilement déterminés par l'homme de métier en fonction des conditions optimales d'activité de l'enzyme.

Après inactivation de l'enzyme, qui peut être réalisée à 100°C pendant 10 minutes, le produit obtenu sera éventuellement soumis à une ultrafiltration tangantielle de 50000 Daltons, puis à une étape de dessalage sur résine échangeuse d'ions et éventuellement à une réduction sous forme de poudre par lyophilisation ou atomisation.

D'une façon générale, la laminarine et les oligosaccharides dérivés de laminarine utilisés comme agent stimulant, régénérant, revitalisant et à activité énergétique sur les fibroblastes et les kératinocytes dans le cadre de la présente invention, permettent la fabrication de compositions cosmétiques.

Selon une caractéristique particulière, cette composition cosmétique comprend, exprimé en pourcentage en poids, de 0,00001 % à 10 % de laminarine ou d'oligosaccharides dérivés de laminarine.

Avantageusement, cette quantité sera comprise entre 0,1 % et 5 %.

Une telle composition cosmétique peut se présenter sous forme de solution aqueuse, d'émulsion simple ou multiple, ou encore sous forme solide, notamment de poudre, de comprimé ou de gélule.

Ces compositions seront préparées selon les méthodes habituellement utilisées dans les domaines cosmétique ou pharmaceutique.

Par exemple, la laminarine ou les oligosaccharides dérivés de laminarine pourront être dissous dans une phase aqueuse pour la préparation d'une solution aqueuse.

Cette solution pourra éventuellement permettre l'obtention d'une émulsion par mélange avec différents composants tels que des tensioactifs ou émulsionnants, des huiles ou acides gras, des alcools gras, des cires, des gélifiants, des humectants, de la glycérine ou des glycols.

D'une manière facultative, ces compositions pourront en outre comprendre une substance additionnelle choisie parmi les conservateurs et les parfums.

Selon un deuxième aspect, la présente demande vise à couvrir l'utilisation de la laminarine ou d'oligosaccharides dérivés de laminarine pour la fabrication d'un médicament destiné au traitement de la peau.

Selon une caractéristique particulière, ce médicament se présentera sous forme solide, notamment de poudre, de comprimé, de gélule ou de sirop buvable ou de crème à application topique.

Ces formes pharmaceutiques seront préparées selon les méthodes usuelles.

Le principe actif constitué par une quantité pharmaceutiquement efficace de laminarine ou d'oligosaccharides dérivés de laminarine, sera incorporé à des excipients habituellement employés dans ces compositions pharmaceutiques, tels que le talc, la gomme arabique, le lactose, l'amidon, le stéarate de magnésium, la polyvidonne, les dérivés de la cellulose, des corps gras d'origine animale ou végétale, et divers agents mouillants, dispersants ou émulsifiants ainsi que des conservateurs, aromes et colorants.

Selon un troisième aspect, la présente demande vise à couvrir une composition cosmétique ou pharmaceutique, notamment dermatologique, caractérisée en ce qu'elle comprend, à titre d'ingrédient actif, une quantité efficace d'oligosaccharides dérivés de laminarine, en association avec un support ou véhicule cosmétiquement ou pharmaceutiquement acceptable.

Selon une caractéristique particulière, cette composition cosmétique comprend, exprimé en pourcentage en poids, de 0,00001 % à 10 % d'oligosaccharides dérivés de laminarine.

Avantageusement, cette quantité sera comprise entre 0,1 % et 5 % en poids.

Une telle composition cosmétique peut se présenter sous forme de solution aqueuse, d'émulsion simple ou multiple, ou encore sous forme solide, notamment de poudre, de comprimé ou de gélule.

Cette composition peut être préparée selon les méthodes habituellement utilisées dans les domaines cosmétiques ou pharmaceutiques et rappelées précédemment

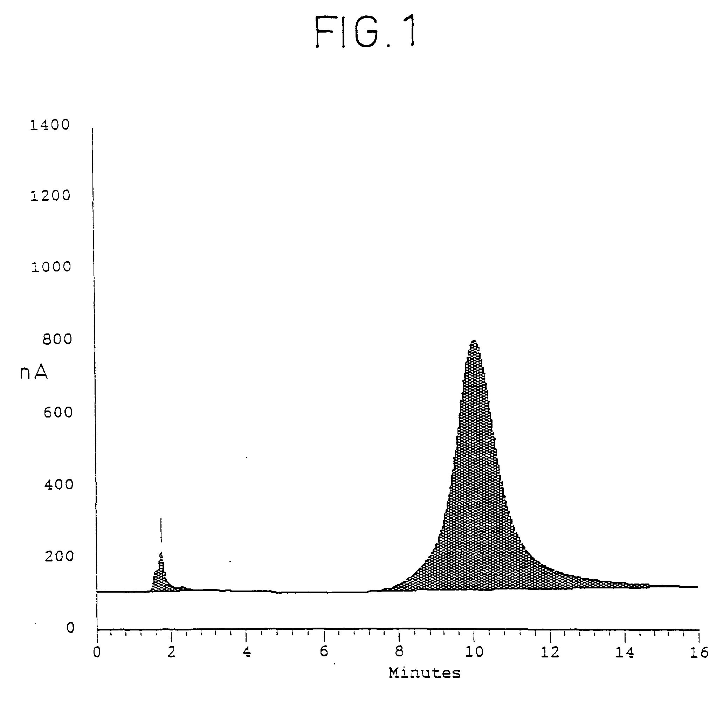

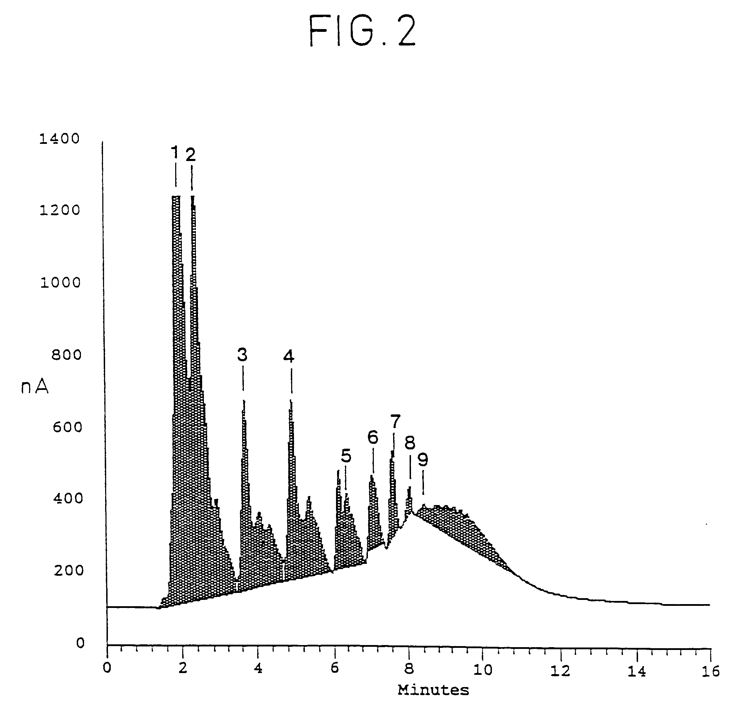

Il est fait référence aux figures 1 et 2.

La présente invention sera illustrée plus en détail à l'aide des exemples non limitatifs suivants.

La laminarine peut être extraite, conformément au procédé mentionné dans la demande de brevet français No. 92.08387. Les exemples 1 et 2 ci-après illustrent d'autres procédés d'obtention de laminarine ou d'oligosaccharides dérivés de laminarine.

### EXEMPLE 1

### Procédé d'extraction de la laminarine à partir d'une algue brune.

On soumet 105 g d'algues sèches de type Laminaria digitata à une extraction par 1,5 L d'acide sulfurique 0,09 M.

L'extraction est réalisée au bain-marie à une température d'environ 70°C pendant 2H30.

L'extrait est filtré sous vide sur un filtre Whatman GF/A, puis sur un filtre Millipore à 0.45 µm.

Le liquide ainsi obtenu est soumis à une ultrafiltration tangentielle à 5000 Daltons. A cet effet, on a utilisé un système Pellicon commercialisé par la Société MILLIPORE, équipé d'une cassette de porosité 5000 Daltons, associé à une pompe Procon également commercialisée par la Société MILLIPORE.

L'opération est réalisée en maintenant une pression de 3 bars sur la cassette de filtration.

On obtient ainsi un filtrat d'environ 1,3 litre et de pH 3 à 5, qui est ensuite soumis à une lyophilisation pour conduire à 5,5 g de poudre sèche correspondant à la laminarine pure à 90%.

### EXEMPLE 2

### Procédé de préparation d'oligosaccharides dérivés de laminarine.

On soumet 10 g de laminarine extraite selon l'exemple 1 à l'action de 1 g d'une β(1-3) glucanase (laminarinase). La réaction enzymatique est réalisée dans 1 L de milieu tampon acétate 0,05 M de pH 5.

On met ce mélange à incuber à 37°C pendant 20 minutes. Puis on effectue une inactivation de l'enzyme au bain-marie à 100°C pendant 10 minutes. Après refroidissement à température ambiante, le produit est soumis à une ultrafiltration tangentielle à 50000 Daltons de manière à éliminer l'enzyme inactivée.

Ceci est réalisé sur une membrane tubulaire carbone-céramique de type "Carbosep" de porosité 50000 Daltons.

L'opération est réalisée en maintenant une pression de 1 bar sur la colonne de filtration. On obtient ainsi un filtrat présentant un volume d'environ 0,9 litre, de pH 5 et de résistivité = 175 Ohms/cm.

Celui-ci est ensuite soumis à une étape de dessalage. A cet effet, il est traité sur une colonne de résine échangeuse de cations de type échangeur fort tel que Amberlite IR 120® puis, sur une colonne de résine échangeuse d'anions de type échangeur fort tel que Amberlite IRA 400®.

On obtient ainsi un produit présentant une résistivité de 2000 Ohms/cm, de volume 0,9 L. Après atomisation, on obtient 3,6 g d'oligosaccharides dérivés de laminarine.

La laminarine et les oligosaccharides dérivés de laminarine obtenus aux exemples 1 et 2 ont été soumis à une chromatographie en phase liquide à haute performance.

Les spectres HPLC obtenus sont donnés respectivement aux figures 1 et 2.

Il est précisé que ces spectres ont été obtenus avec une injection de 50 µl en utilisant un chromatographe DIONEX® et un ampéromètre comme détecteur.

### EXEMPLE 3

Les propriétés des produits conformes à la présente invention ont été mises en évidence en effectuant les expériences décrites ci-après.

### EXPERIENCE No. 1

### Mise en évidence des propriétés stimulantes de la laminarine dans des cultures de fibroblastes du derme humain.

L'effet de la laminarine obtenue selon l'exemple 1 a été étudié dans des cultures de fibroblastes de derme humain. L'activité de la laminarine a été évaluée après 20 heures d'incubation par la mesure d'un paramètre fonctionnel, l'incorporation de Leucine radiomarquée au carbone 14 dans les protéines, reflet de la néosynthèse des protéines.

Les fibroblastes ont été isolés à partir de prélèvements de peau effectués lors de plasties mammaires sur des femmes âgées de 44 ans. Ces fibroblastes ont été obtenus après dissociation de l'épiderme et du derme par la trypsine,

Des cultures témoins ont été effectuées en parallèle, en l'absence de laminarine :
⇒ soit dans le milieu "culture-témoin" :
   - MCIF : milieu de culture pour l'incubation des fibroblastes en présence de milieu MEM (milieu minimum essentiel) additionné de 100 Ul/ml de pénicilline et 100 µg/ml de streptomycine.
⇒ soit dans le milieu "culture-témoin positif" :
   - MCAF milieu de culture pour l'attachement des fibroblastes : milieu MEM/M199 (appellation donnée à un milieu de culture standard) dans les proportions 3/4 : 1/4 (V/V) additionné de 100 Ul/ml de pénicilline, de 100 µ g/ml de streptomycine et de 10% (V/V/) de sérum de veau foetal (SVF).

Le milieu complet (témoin positif) provoque une augmentation de l'incorporation de la leucine dans les protéines de fibroblastes d'un facteur égal à 1.7.

Cette valeur se situe dans la gamme de celles habituellement obtenues au laboratoire ; elle démontre la capacité des cellules à répondre à des facteurs stimulants.

La laminarine obtenue selon l'exemple 1 a été mise en présence des fibroblastes en cours de prolifération pendant 20 heures.

Les fibroblastes ont été incubés avec la leucine radiomarquée au ¹⁴C (marquage au ¹⁴C du carbone 1, activité spécifique 1.92 GBq/mmole) pendant 4 heures. Le milieu de culture utilisé pour l'incorporation de ¹⁴C leucine dans les protéines est le milieu MEM sans leucine additionné de 100 Ul/ml de pénicilline, de 100 µg/ml de streptomycine et de 9.6 10⁻⁶M de leucine radiomarquée (18.5 kBq/ml). Les tapis cellulaires ont été lavés afin d'éliminer le précurseur non incorporé puis comptés en scintillation liquide. Les valeurs ont été exprimées en dpm/puits de culture.

La laminarine est directement solubilisée dans le milieu d'incubation à des doses de 0.1, 1, 10, 100 et 1000 µg/ml. Chaque essai est répété 4 fois.

Les résultats obtenus sont regroupés dans le tableau suivant :

| | Témoin sans laminarine | LAMINARINE EN µg/ml | | | | |
|---|---|---|---|---|---|---|
| | | 0.1 | 1 | 10 | 100 | 1000 |
| Essai 1 | 818 | 872 | 877 | 1015 | 979 | 978 |
| Essai 2 | 761 | 801 | 976 | 1188 | 1108 | 1070 |
| Essai 3 | 739 | 848 | 926 | 973 | 980 | 974 |
| Essai 4 | 769 | 875 | 888 | 1070 | 932 | 1053 |
| Moyenne ± Ecart type | 772 ± 33 | 849 ± 34 | 917 ± 45 | 1062 ± 93 | 1000 ± 76 | 1019 ± 50 |
| % par rapport au témoin | 100 | 110 | 119 | 138 | 130 | 132 |

La laminarine présente, dans les conditions expérimentales testées, une activité optimale à la concentration de 10 µg/ml.

La laminarine augmente la néosynthèse des protéines dans les fibroblastes, d'un facteur 1,3 aux concentrations comprises entre 10 et 1000 µg/ml, dans un milieu de culture totalement dépourvu de sérum de veau foetal, ce qui démontre l'effet stimulant de la laminarine.

### EXPERIENCE No. 2

### Mise en évidence des propriétés stimulantes de la laminarine dans des cultures de kératinocytes de l'épiderme humain.

L'effet de la laminarine obtenue selon l'exemple 1 a été étudiée dans des cultures de kératinocytes d'épiderme humain.

Son activité a été évaluée après 20 heures d'incubation par la mesure d'un paramètre fonctionnel, l'incorporation de leucine radiomarquée au carbone 14 dans les protéines, reflet de la néosynthèse des protéines.

Les kératinocytes ont été isolés à partir de prélèvements de peau effectués lors de plasties mammaires sur des femmes âgées de 34 ans. Ces kératinocytes ont été obtenus après dissociation de l'épiderme et du derme par la trypsine, ils ont été cultivés avec des fibroblastes d'embryons de souris (3T3) dont la capacité à se diviser a été bloquée par la mitomycine C (modèle de Green).

Des cultures témoins ont été effectuées en parallèle, en l'absence de laminarine:
⇒ soit dans le milieu "culture témoin" (MCIK) milieu de culture pour l'incubation des kératinocytes en présence des composés MCAK, milieu sans facteur de croissance épidermique EGF, ni sérum de veau foetal SVF, dilué au 1/10e dans du milieu MEM (Milieu minimum essentiel), additionné de 100 Ul/ml de pénicilline et de 100 µg/ml de streptomycine.
⇒ Soit dans le milieu "culture témoin positif" (MCAK) : milieu de culture pour l'attachement des kératinocytes c'est-à-dire un milieu MEM/M199 (appellation donnée à un milieu de culture standard) dans les proportions 3/4 : 1/4 (V/V) additionné de 100 Ul/ml de pénicilline, de 100 µ g/ml de streptomycine, de 10% (V/V) de sérum de veau foetal, de 10 ng/ml de facteur de croissance épidermique (EGF) de 10 ng/ml de toxine cholérique, de 5 µg/ml d'insuline bovine, de 0,4 µg/ml d'hydrocortisone, de 5 µg/ml de choline et de 8,5 µg/ml d'inositol.

La laminarine a été mise en présence des kératinocytes en cours de prolifération pendant 20 heures.

Les kératinocytes ont été incubés avec la leucine radiomarquée au ¹⁴C pendant 4 heures. Le milieu de culture utilisé pour l'incorporation de ¹⁴C leucine dans les protéines est le milieu MEM sans leucine additionné de 100 Ul/ml de pénicilline, de 100 µg/ml de streptomycine et de 9.6 10⁻⁶M de leucine radiomarquée (18.5 kBq/ml).

Les tapis cellulaires ont été lavés afin d'éliminer le précurseur non incorporé puis comptés en scintillation liquide. Les valeurs ont été exprimées en dpm/puits de culture.

La laminarine est directement solubilisée dans le milieu d'incubation à des doses de 0.1, 1, 10, 100, 1000 µg/ml. Chaque essai est répété 4 fois.

Les résultats obtenus sont regroupés dans le tableau suivant:

| | Témoin sans laminarine | LAMINARINE EN *µ*g/ml | | | | |
|---|---|---|---|---|---|---|
| | | 0.1 | 1 | 10 | 100 | 1000 |
| Essai 1 | 5862 | 6033 | 6522 | 7462 | 7693 | 6264 |
| Essai 2 | 5642 | 6733 | 7178 | 8159 | 8778 | 7723 |
| Essai 3 | 5642 | 5869 | 7449 | 9909 | 7713 | 7386 |
| Essai 4 | 5375 | 4966 | 5648 | 8241 | 7073 | 7811 |
| Moyenne ± Ecart type | 5630 ± 199 | 5900 ± 727 | 6699 ± 802 | 8443 ± 1038 | 7814 ± 708 | 7296 ± 712 |
| % par rapport au témoin | 100 | 105 | 119 | 150 | 139 | 130 |

Ainsi, la laminarine augmente la néosynthèse des protéines dans les kératinocytes du derme humain, d'un facteur de 1,3 à 1,5 aux concentrations comprises entre 10 et 1000 µg/ml. Elle présente, dans les conditions expérimentales testées, une activité optimale à la concentration de 10 µg/ml.

### EXPERIENCE No. 3

### Mise en évidence des propriétés stimulantes des oligosaccharides dérivés de la laminarine dans des cultures de fibroblastes du derme humain.

Dans cette expérience, l'effet étudié est celui des oligosaccharides dérivés de la laminarine obtenus selon l'exemple 2.

Les conditions opératoires de cette expérience sont les mêmes que celles de l'expérience No. 1. Les oligosaccharides sont directement solubilisés dans le milieu d'incubation à des doses de 0.1, 1, 10, 100, 1000 µg/ml. Chaque essai est répété 4 fois.

Les résultats obtenus sont regroupés dans le tableau suivant :

| | Témoin sans Oligosacch. | OLIGOSACCHARIDES DERIVES DE LA LAMINARINE (µg/ml) | | | | |
|---|---|---|---|---|---|---|
| | | 0.1 | 1 | 10 | 100 | 1000 |
| Essai 1 | 818 | 798 | 861 | 1022 | 1014 | 1059 |
| Essai 2 | 761 | 925 | 947 | 1074 | 1169 | 958 |
| Essai 3 | 739 | 846 | 860 | 1146 | 1184 | 984 |
| Essai 4 | 769 | 806 | 781 | 1163 | 1154 | 1129 |
| Moyenne ± Ecart type | 772 ± 33 | 844 ± 58 | 862 ± 68 | 1101 ± 65 | 1130 ± 78 | 1032 ± 77 |
| % par rapport au témoin | 100 | 109 | 112 | 143 | 146 | 134 |

Ces résultats montrent que les oligosaccharides dérivés de la laminarine augmentent la néosynthèse des protéines dans les fibroblastes du derme humain d'un facteur 1.4 aux concentrations comprises entre 10 et 1000 µg/ml. Les effets maximaux sont observés à 10 et 100 µg/ml.

### EXPERIENCE No. 4

### Mise en évidence des propriétés stimulantes des oligosaccharides dérivés de la laminarine dans des cultures de kératinocytes de l'épiderme humain.

Dans cette expérience, l'effet étudié est celui des oligosaccharides dérivés de la laminarine obtenus selon l'exemple 2.

Les conditions opératoires de cette expérience sont les mêmes que celles de l'expérience No. 2. Les oligosaccharides sont directement solubilisés dans le milieu d'incubation à des doses de 0.1, 1, 10, 100, 1000 µg/ml. Chaque essai est répété 4 fois.

Les résultats obtenus sont regroupés dans le tableau suivant :

| | Témoin sans oligosacch. | Oligosaccharides dérivés de laminarine (*µ*g/ml) | | | | |
|---|---|---|---|---|---|---|
| | | 0.1 | 1 | 10 | 100 | 1000 |
| Essai 1 | 5862 | 6652 | 7075 | 8124 | 9028 | 7503 |
| Essai 2 | 5642 | 6551 | 7281 | 8289 | 9314 | 9181 |
| Essai 3 | 5642 | 6701 | 7361 | 8862 | 9483 | 8611 |
| Essai 4 | 5375 | 5627 | 6379 | 9973 | 8634 | 7697 |
| Moyenne ± Ecart type | 5630 ± 199 | 6383 ± 508 | 7024 ± 447 | 8812 ± 836 | 9115 ± 371 | 8248 ± 788 |
| % par rapport au témoin | 100 | 113 | 125 | 157 | 162 | 146 |

Ces résultats montrent que les oligosaccharides dérivés de la laminarine augmentent la néosynthèse des protéines dans les kératinocytes de l'épiderme humain d'un facteur 1.6 aux concentrations comprises entre 1 et 1000 µg/ml. Les effets maximaux sont observés à 10 et 100 µg/ml.

On donnera aux exemples 4 à 7 ci-après différentes compositions cosmétiques ou pharmaceutiques contenant de la laminarine ou des oligosaccharides dérivés de la laminarine tels que préparés aux exemples 1 et 2.

### EXEMPLE 4

### Formulation d'une pommade pharmaceutique à action cicatrisante dans laquelle on intègre la laminarine.

| POMMADE CICATRISANTE | |
|---|---|
| | % |
| Huile de paraffine | 95.1 |
| Trioléate de sorbitan polyoxyéthylène (200E) | 2.5 |
| Extrait de Calendula | 1.0 |
| Huile essentielle de Melaleuca Alternifolia | 0.5 |
| Laminarine | 0.5 |
| Conservateur | 0.4 |

### EXEMPLE 5

### Formulation d'une crème cosmétique à action régénérante contenant de la laminarine comme principe actif.

| CREME REGENERANTE | |
|---|---|
| | % |
| Eau purifiée | 69.4 |
| Stéarate d'isocéthyl | 11.0 |
| Extrait d'algues | 6.0 |
| Alcool cétylique | 4.0 |
| Stéarate de glycérol | 3.5 |
| Ether de polyéthylène glycol du stéarate de glycérol | 3.5 |
| Huile de silicone | 1.0 |
| Laminarine | 0.6 |
| Sodium pyrrolidone carboxylate | 0.5 |
| Conservateur | 0.3 |
| Parfum | 0.2 |

### EXEMPLE 6

### Formulation d'une lotion hydratante contenant les gligosaccharides dérivés de laminarine comme principe actif.

| LOTION HYDRATANTE | |
|---|---|
| | % |
| Eau | 89.1 |
| Extrait d'algues | 8.0 |
| Glycerine | 1.5 |
| Oligosaccharides dérivés de Laminarine préparés selon l'exemple 2 | 1.0 |
| Conservateur | 0.3 |
| Parfum | 0.1 |

### EXEMPLE 7

### Formulation d'un dentifrice marin contenant les oligosaccharides dérivés de la laminarine comme principe actif.

| DENTIFRICE MARIN | |
|---|---|
| | % |
| Eau | 47.6 |
| Carbonate de calcium | 25.0 |
| Algue rouge | 12.0 |
| Extrait d'algue | 10.0 |
| Carrageenan | 2.0 |
| Oligosaccharides de laminarine préparés selon l'exemple 2 | 1.0 |
| Silice | 0.8 |
| Lauryléther sulfate de sodium | 0.8 |
| Methylparaben | 0.5 |
| Arôme | 0.3 |

## Revendications

1. Utilisation en cosmétologie de la laminarine et des oligosaccharides dérivés de laminarine comme agent stimulant, régénérant, revitalisant et à activité énergétique sur les fibroblastes et les kératinocytes.

2. Utilisation selon la revendication 1, dans une composition comprenant, exprimée en pourcentage en poids, de 0,00001 % à 10 % de laminarine ou d'oligosaccharides dérivés de laminarine.

3. Utilisation selon la revendication 1, dans une composition cosmétique comprenant, exprimée en pourcentage en poids, entre 0,1 % et 5 % de laminarine ou d'oligosaccharides dérivés de laminarine.

4. Utilisation selon l'une des revendications 1 à 3, dans une composition cosmétique se présentant sous forme de solution aqueuse, d'émulsion simple ou multiple.

5. Utilisation de la laminarine ou d'oligosaccharides dérivés de laminarine pour la fabrication d'un médicament destiné au traitement de la peau.

6. Utilisation selon la revendication 5, caractérisée en ce que le médicament précité se présente sous forme solide, notamment de poudre, de comprimé, de gélule ou de sirop buvable ou de crème à application topique.

7. Composition cosmétique ou pharmaceutique, notamment dermatologique, caractérisée en ce qu'elle comprend à titre d'ingrédient actif, une quantité efficace d'oligosaccharides dérivés de laminarine, en association avec un support ou véhicule cosmétiquement ou pharmaceutiquement acceptable.

8. Composition selon la revendication 7, caractérisée en ce qu'elle comprend, exprimé en pourcentage en poids, de 0,00001 % à 10 % d'oligosaccharides dérivés de laminarine.

9. Composition selon la revendication 8, caractérisée en ce qu'elle comprend, exprimé en pourcentage en poids entre 0,1 % et 5% d'oligosaccharides dérivés de laminarine.

10. Composition selon l'une des revendications 7 à 9, caractérisée en ce qu'elle se présente sous forme de solution aqueuse, d'émulsion simple ou multiple, ou encore sous forme solide, notamment de poudre, de comprimé ou de gélule.

## Patentansprüche

1. Verwendung von Laminarin und von Laminarin abgeleiteten Oligosacchariden als hinsichtlich Fibroblasten und Keratinozyten stimulierendes, regenerierendes, revitalisierendes und eine kraftspendende Aktivität aufweisendes Mittel in der Kosmetik.

2. Verwendung nach Anspruch 1 einer Zusammensetzung, die, ausgedrückt in Gewichtsprozent, 0,00001% bis 10% Laminarin oder von Laminarin abgeleitete Oligosaccharide umfasst.

3. Verwendung nach Anspruch 1 einer kosmetischen Zusammensetzung, die, ausgedrückt in Gewichtsprozent, zwischen 0,1% und 5% Laminarin oder von Laminarin abgeleitete Oligosaccharide umfasst.

4. Verwendung nach einem der Ansprüche 1 bis 3 einer kosmetischen Zusammensetzung, die in Form einer wässrigen Lösung, einer einfachen oder mehrfachen Emulsion vorliegt.

5. Verwendung von Laminarin oder von Laminarin abgeleiteten Oligosacchariden für die Herstellung eines Arzneimittels, das für die Behandlung der Haut bestimmt ist.

6. Verwendung nach Anspruch 5, dadurch gekennzeichnet, dass das vorerwähnte Arzneimittel in fester Form, insbesondere eines Pulvers, einer Tablette, einer Kapsel, oder eines trinkbaren Sirups oder einer Creme für eine topische Anwendung vorliegt.

7. Kosmetische oder pharmazeutische, insbesondere dermatologische Zusammensetzung, dadurch gekennzeichnet, dass sie als Wirkstoff eine wirksame Menge von von Laminarin abgeleiteten Oligosacchariden in Verbindung mit einem kosmetisch oder pharmazeutisch annehmbaren Träger oder Vehikel umfasst.

8. Zusammensetzung nach Anspruch 7, dadurch gekennzeichnet, dass sie, ausgedrückt in Gewichtsprozent, 0,00001% bis 10% von Laminarin abgeleitete Oligosaccharide umfasst.

9. Zusammensetzung nach Anspruch 8, dadurch gekennzeichnet, dass sie, ausgedrückt in Gewichtsprozent, zwischen 0,1% und 5% von Laminarin abgeleitete Oligosaccharide umfasst.

10. Zusammensetzung nach einem der Ansprüche 7 bis 9, dadurch gekennzeichnet, dass sie in Form einer wässrigen Lösung, einer einfachen oder mehrfachen Emulsion oder ferner in fester Form, insbesondere eines Pulvers, einer Tablette oder einer Kapsel, vorliegt.

## Claims

1. Use in cosmetology of laminarin and oligosaccharides derived from laminarin as a stimulating agent, a regenerating agent, a revitalizing agent and an agent having energizing activity on the fibroblasts and the keratinocytes.

2. Use according to claim 1, of a composition containing, expressed in percentage by weight, from 0.00001% to 10% of laminarin or oligosaccharides derived from laminarin.

3. Use according to claim 1, of a cosmetic composition containing, expressed in percentage by weight, between 0.1% and 5% of laminarin or oligosaccharides derived from laminarin.

4. Use according to claims 1 to 3, of a cosmetic composition in the form of an aqueous solution, or a simple or multiple emulsion.

5. Use of laminarin or oligosaccharides derived from laminarin for the manufacture of a medicine destined for the treatment of the skin.

6. Use according to claim 5, characterised in that the above-mentioned medicine is in the form of a solid, notably a powder, a tablet, a gelatine capsule or a drinkable syrup, or cream for topical application.

7. A cosmetic or pharmaceutical composition, notably a dermatological composition, characterised in that it includes as active ingredient, an effective amount of oligosaccharides derived from laminarin, in combination with a cosmetically or pharmaceutically acceptable carrier or vehicle.

8. The composition according to claim 7, characterised in that it contains, expressed in percentage by weight, from 0.00001% to 10% of oligosaccharides derived from laminarin.

9. The composition according to claim 8, characterised in that it contains, expressed in percentage by weight, between 0.1% and 5% of oligosaccharides derived from laminarin.

10. The composition according to one of claims 7 to 9, characterised in that it is in the form of an aqueous solution, a simple or multiple emulsion, or even in the form of a solid, notably a powder, a tablet or a gelatine capsule.
